# EUROPEAN PATENT APPLICATION

(11) **EP 3 037 111 A1**
(43) Date of publication of application: **29.06.2016**
(21) Application number: 15382659.9
(22) Date of filing: 23.12.2015
(51) Int. Cl.: A61L 12/08, A45C 11/00, A45C 11/04

(54) **CONTACT LENS CASE FOR CLEANING THEREOF**

(30) Priority: 23.12.2014 ES 201431932
(71) Applicant: DISOP.S.A., 28108 Alcobendas (Madrid) (ES)
(72) Inventor: GARCIA-BLANES DIAZ, Enrique, 28108 ALCOBENDAS (Madrid) (ES); ARRANZ AGUIRRE, Juan, 28108 ALCOBENDAS (Madrid) (ES)
(74) Representative: Carpintero Lopez, Francisco

(57) **Abstract**

The invention enables maximised cleaning of contact lenses, guaranteeing hermetic and effective sealing of the lens case (1), wherein each of the lids (30) comprises a central recess (31) and a protuberance (32), wherein said protuberances (32) are dimensionally adapted for firmly coupling with and fitting in each of the cavities (11) of the base body (10); wherein in the closed position of the lids (30), each assembly formed by a cavity (11), recess (31) and protuberance (32) shares the same imaginary central vertical axis (I); and where at least the cavities (11) and protuberances (32) are made of flexible material, such that in the closed position of the lids (30) the contact surface between the cavities (11) and the protuberances (32) is elastically deformable by application of rubbing and/or pressure thereon, in order to achieve optimum cleaning of the contact lenses.

## Description

### OBJECT OF THE INVENTION

The present invention belongs to the field of optics and, more specifically, to the manufacture of products for the care and maintenance of contact lenses.

The object of the present invention is a lens cases specially configured to enable effective, quick and simple cleaning of contact lenses, while guaranteeing a hermetic and effective seal, without independent parts or lids that can fall or become lost, and without threaded closing mechanisms, thereby avoiding unwanted spillage of liquid.

### BACKGROUND OF THE INVENTION

Currently, different types of contact lens cases or holders are widely known, appropriate for storing, transporting and maintaining the lenses inside a contact lens solution or liquid. Of all these, the most widespread is probably that having a pair of receptacles made of rigid plastic material with external threading and a pair of independent lids having, in turn, internal threading for firmly closing the case by means of a threaded joint.

Thus, various drawbacks have been detected in current contact lens cases or holders, namely:
- Their closing mechanisms are based on parts or lids independent from the main body of the case, said lids being susceptible to becoming lost or falling on the ground, with the ensuing problems, such as spillage of the liquid or contagion of bacteria or other pathogenic microorganisms harmful to the eyes.
- The closing mechanisms of the preceding point require external threading in the receptacles and internal threading in the lids, rendering the operation slow and cumbersome. Furthermore, on not forming part of the lens case, they are easy to confuse or lose. This threaded joint accumulates dirt, particles and germs, since its configuration and design are not completely aseptic.

Japanese patent JP2009109892A is known, which discloses a contact lens cleaning container that includes a "bag" having in turn an inlet mouth wherethrough the lenses and a cleaning liquid are introduced. This invention, while focusing on the cleaning of lenses, has various drawbacks, namely:
- it requires a large amount of cleaning liquid to fill the bag;
- the bag can be pierced, ripped or penetrated by any sharp element, with the ensuing spillage of liquid and ineffectiveness of the container;
- it is difficult and cumbersome to find and extract the lenses once cleaned, as they remain inside the bag adhered to its inner sides.

### DESCRIPTION OF THE INVENTION

The present invention solves the aforementioned drawbacks, providing a contact lens case which makes it possible to optimise lens cleaning in a quick, simple and effective way, while guaranteeing hermetic and effective sealing thereof, without independent parts or lids that can fall or become lost, and without threaded sealing mechanisms, avoiding the accumulation of dirt which favours the establishment and proliferation of microorganisms.

The lens case comprises a base body having in turn two cavities for housing a cleaning solution or liquid in their interior; a pair of hinge elements fixed to the base body; and a pair of lids joined to the hinge elements, swinging between an open and closed position and vice versa.

More particularly, each of the lids of the lens case of the invention comprises in turn a central recess on its outer side and a protuberance on its inner side, wherein said protuberances are dimensionally adapted for firmly coupling to and fitting in each of the cavities of the base body. In the present invention, "outer side" of the lids shall be understood to be the surface which remains visible and exposed to the exterior in the closed position. Furthermore, "inner side" shall be understood to be the surface of the lids that remains concealed in the closed position.

In addition, in the closed position of the lids, each assembly formed by a cavity, recess and protuberance shares a common imaginary central vertical axis. This enables perfect coupling and positioning of the protuberances of the lids in the interior of the cavities of the base body, which favours optimum cleaning of the contact lenses.

Likewise, at least the cavities of the base body and the protuberances of the lids are made of flexible, non-rigid material, preferably manufactured from a flexible elastomer, such that in the closed position of the lids the contact surface between the cavities and the protuberances is elastically deformable by application of rubbing and/or pressure thereon. This allows the user to clean his or her contact lenses effectively and quickly with a simple gesture, by just introducing one of his or her fingers in the lid recesses and slightly rubbing or exerting pressure on the surface of the lids against the cavities of the base body. Although said rubbing process can be performed as the user desires, ideally the use of two fingers has been envisaged, the thumb for the lower side of the cavities and the index finger for insertion in the lid recesses.

Also, the possibility is envisaged that both the cavities of the base body and the protuberances of the lids may have projections, preferably circular and concentric, disposed on the surface thereof. These projections favour and maximise the cleaning and hygiene of the contact lenses in the closed position of the lids. In fact, the friction and rubbing of the projections of each protuberance against the projections of each cavity enable the contact lens disposed therebetween to be fully and effectively cleaned on both sides, enabling the removal of any particle of dust, dirt or residue that it may contain.

Likewise, in accordance with a preferred embodiment, the lens case object of the invention has been envisaged to additionally have closing and opening means that comprise at least one lateral notch, made in the central zone of the base body, between the two cavities, wherein said notch has in turn at least one pair of hollow countersinks; and at least one action surface disposed in each of the lids and which in turn has at least one flexible tab for being firmly inserted and fitted in the hollow countersinks of the lateral notch of the base body.

### DESCRIPTION OF THE DRAWINGS

As a complement to the description made below, and for the purpose of helping to make the characteristics of the invention more readily understandable, in accordance with a preferred embodiment thereof, this specification is accompanied by a set of drawings that form an integral part thereof and whose figures, by way of illustration and not limitation, represent the following:
Figure 1 shows a perspective view of the lens case of the invention in the closed position.
Figure 2 shows another perspective view of the lens case in the open position.

### PREFERRED EMBODIMENT OF THE INVENTION

What follows is a preferred embodiment making reference to the two aforementioned figures, without limiting or reducing the scope of protection of the present invention.

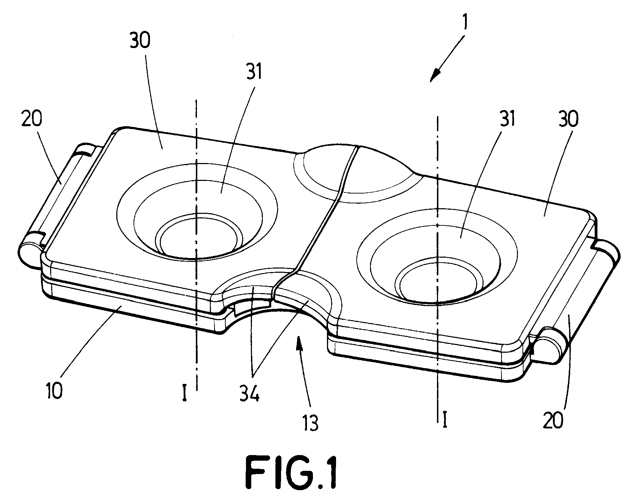

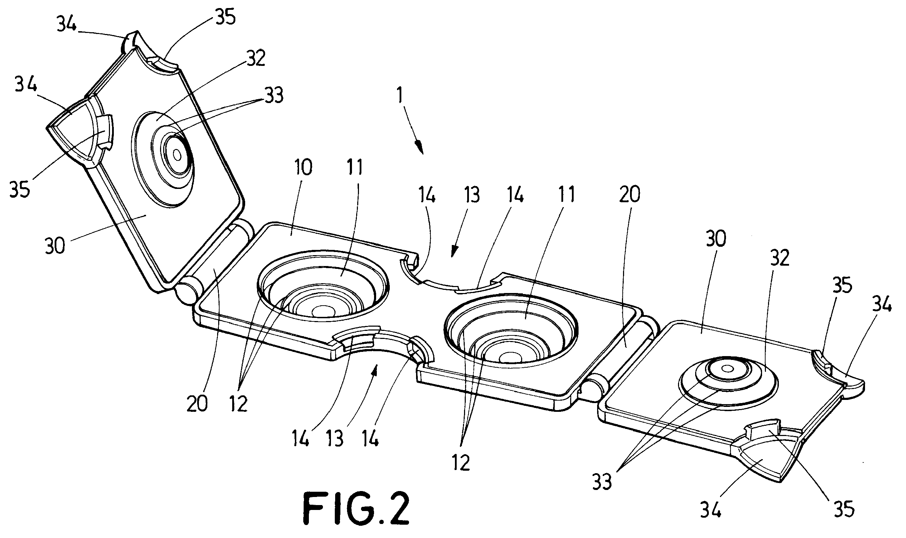

Figure 1 shows a possible example of the contact lens of the invention, with its lids in the closed position. More specifically, the lens case (1) shown herein is of the type comprising a base body (10) having in turn two cavities (11), shown in figure 2, destined for housing a cleaning solution or liquid in their interior; a pair of hinge elements (20) fixed to the base body (10); and a pair of lids (30) joined to the hinge elements (20), swinging between an open and closed position and vice versa.

As can be observed in said figure 1, each of the lids (30) comprises in turn a central recess (31) on its outer side and a protuberance (32) on its inner side, wherein said protuberances (32), shown in figure 2, are dimensionally adapted for firmly coupling to and fitting in each of the cavities (11) of the base body (10).

Thus, in the closed position of the lids (30), shown in figure 1, each assembly formed by a cavity (11), recess (31) and protuberance (32) shares the same imaginary central vertical axis (I), which allows for adequate positioning and coupling between the protuberances (32) and the cavities (11).

In the present embodiment, the cavities (11) of the base body (10) and the protuberances (32) of the lids (30) are made of flexible elastomer, such that in the closed position of the lids (30), the contact surface between the cavities (11) and the protuberances (32) is elastically deformable due to the application of rubbing and/or pressure thereon. Said elastic deformation makes it possible to cover and sweep the entire lens surface, moving the internal lens liquid and removing any minimum particle of dust or residue.

Furthermore, figure 2 shows that both the cavities (11) of the base body (10) and the protuberances (32) of the lids (30) have projections (12, 33) with a circular and concentric configuration, disposed on its surface for maximising cleaning of the contact lens in the closed position of the lids (30). Thus, the rubbing of the users' fingers causes the projections (12, 33) to move slightly and extend throughout the surface of the contact lenses, removing and dragging away any dirt, micro-particle or residue they may present. Notwithstanding the foregoing, said projections have been envisaged (12, 33) to have a similar shape to that of a person's fingerprints, further maximising the cleaning of the contact lenses.

In figures 1 and 2, it can be observed that the cavities (11) of the base body (10) and the recesses (31) of the lids (30) have a concave shape, while the protuberances have a convex shape. This allows optimum positioning of the lens inside each cavity (11), while favouring the firm coupling between each pair formed by protuberance (32) and cavity (11), thereby maximising the complete cleaning of each lens, both on its upper side and on its lower side. To this end, the recesses (31) of the lids (30) have been envisaged to have sufficient diameter to allow for the insertion of a finger by the user.

As represented in figure 2, the base body (10) has a rectangular shape and the hinge elements (20) are disposed in opposition and parallel to each other at opposite ends of the base body (10), such that the lids (30) swing in the same longitudinal direction of the rectangular base body (10). This also allows for a minimum space required for opening and closing the lens case, allowing an opening of 180°.

In the present example, the lens case (1) also includes closing and opening means which comprise two semi-circular lateral notches (13), shown in figure 2, made in the central zone of the base body (10), between the two cavities (11), wherein said notches (13) have in turn a pair of hollow countersinks (14); and two wave-shaped action surfaces (34) disposed on each of the corners of the lids (30), and which in turn have a flexible tab (35) for firmly inserting and fitting in the hollow countersinks (14) of the lateral notches (13) of the base body (10). This closing mechanism of the lids (30) based on the insertion of flexible tabs (35) in hollow countersinks (14) guarantees effective, hermetic and airtight sealing of the lens case (1), wherein each lens remains firmly trapped between the protuberance (32) and the cavity (11), without possibility of movement thereof, and preventing the cleaning liquid from leaking or spilling out to the exterior.

The fact that the action surfaces (34) have a wave-like shape makes it possible for said action surfaces (34) to be disposed in opposition to each other in the closed position of the lids (30), constituting an arched section that projects with respect to the horizontality of the lids (30), as can be observed in figure 1. This makes it possible to favour the opening of the lids (30), insofar as a larger hollow or space is created for the partial insertion of the user's fingers and, therefore, the swinging of each of the lids (30).

Lastly, mention should be made of the main advantages obtained by means of the contact lens case of the invention:
- It maximises the cleaning of the contact lenses by application of rubbing and pressure with the user's own fingers on the lens case.
- Cleaning is performed quickly, simply and effectively by the user, without need for direct contact between the lenses and the hands.
- The existence of internal projections in both the protuberance of the lids and inside the cavities further maximises, if possible, the cleaning of the lenses.
- It provides hermetic and effective sealing of the lens case, without unwanted threaded closures, due to constituting places or recesses where microorganisms thrive, also eliminating any possibility of unwanted dripping, leakage or spillage of the cleaning liquid.
- The lens case of the invention is based on a single piece, not having independent parts or lids that can become lost, fall or be confused.

## Claims

1. Contact lens case (1) for cleaning thereof, which comprises a base body (10) having in turn two cavities (11) for housing cleaning liquid in their interior; a pair of hinge elements (20) fixed to the base body (10); and a pair of lids (30) joined to the hinge elements (20), swinging between an open position and a closed position and vice versa, **characterised in that** each of the lids (30) in turn comprises a central recess (31) on its outer side and a protuberance (32) on its inner side, wherein said protuberances (32) are dimensionally adapted for firmly coupling to and fitting in each of the cavities (11) of the base body (10);
**in that** in the closed position of the lids (30) each assembly formed by a cavity (11), recess (31) and protuberance (32) shares the same imaginary central vertical axis (I); and
**in that** at least the cavities (11) of the base body (10) and the protuberances (32) of the lids (30) are made of a flexible, non-rigid material, such that in the closed position of the lids (30) the contact surface between the cavities (11) and the protuberances (32) is elastically deformable by application of rubbing and/or pressure thereon.

2. Contact lens case (1), in accordance with claim 1, **characterised in that** both the cavities (11) of the base body (10) and the protuberances (32) of the lids (30) have projections (12, 33) disposed on their surface to maximise the cleaning of the contact lenses in the closed position of the lids (30).

3. Contact lens case (1), in accordance with claim 2, **characterised in that** the projections (12, 33) have a circular and concentric configuration.

4. Contact lens case (1), in accordance with claim 1, **characterised in that** the cavities (11) of the base body (10) and the recesses (31) of the lids (30) have a concave shape, while the protuberances (32) have a convex shape, and wherein said recesses (31) have a sufficient diameter to allow the insertion of a finger by a user.

5. Contact lens case (1), in accordance with claim 1, **characterised in that** the base body (10) has a rectangular shape and the hinge elements (20) are disposed in opposition and parallel to each other at opposite ends of the base body (10), such that the lids (30) swing in the same longitudinal direction of the rectangular base body (10).

6. Contact lens case (1), in accordance with claim 1, **characterised in that** it additionally comprises closing and opening means that comprise:
- at least one lateral notch (13), made in the central zone of the base body (10), between the two cavities (11), wherein said notch (13) has in turn at least one pair of hollow countersinks (14); and
- at least one action surface (34) disposed on each of its lids (30) and which in turn has at least one flexible tab (35) for firmly inserting and fitting in the hollow countersinks (14) of the lateral notch (13) of the base body (10).

7. Contact lens case (1), in accordance with claims 5 and 6, **characterised in that** the closing means have two lateral notches (13) in the base body (10) and two action surfaces (34) disposed on each of the corners of the swinging lids (30).

8. Contact lens case (1), in accordance with any one of claims 6 or 7, **characterised in that** the notches (13) are semi-circular.

9. Contact lens case (1), in accordance with any one of claims 6 or 7, **characterised in that** the action surfaces (34) have a wave-like shape, such that in the closed position of the lids (30), said action surfaces (34) are disposed in opposition to each other, constituting an arched section that projects with respect to the horizontality of the lids (30).

10. Contact lens case (1), in accordance with claim 1, **characterised in that** at least the cavities (11) of the base body (10) and the protuberances (32) of the lids (30) are made from flexible elastomer.
